# EUROPÄISCHE PATENTSCHRIFT

(11) **EP 0 603 699 B1**
(45) Veröffentlichungstag und Bekanntmachung des Hinweises auf die Patenterteilung: **15.01.1997**
(21) Anmeldenummer: 93120057.0
(22) Anmeldetag: 13.12.1993
(51) Int. Cl.: C07D 405/06, A61K 31/44, C07D 213/30

(54) **Substituierte Pyridine als HMG-CoA-Reduktase-Inhitoren**
Substituted pyridines as HMG-CoA reductase inhibitors
Pyridines substituées comme inhibiteurs de réductase de HMG-CoA

(30) Priorität: 24.12.1992 DE 4244029
(43) Veröffentlichungstag der Anmeldung: 29.06.1994
(73) Patentinhaber: BAYER AG, 51368 Leverkusen (DE)
(72) Erfinder: Angerbauer, Rolf, Dr., D-42113 Wuppertal (DE); Fey, Peter, Dr., D-42111 Wuppertal (DE); Hübsch, Walter, Dr., D-42113 Wuppertal (DE); Philipps, Thomas, Dr., D-51065 Köln (DE); Bischoff, Hilmar, Dr., D-42113 Wuppertal (DE); Krause, Hans-Peter, Dr., D-58332 Schwelm (DE); Petersen von Gehr, Jörg, Dr., D-44795 Bochum (DE); Schmidt, Delf, Dr., D-42113 Wuppertal (DE)

(56) Entgegenhaltungen:
- EP-A- 0 306 929
- EP-A- 0 325 130
- JOURNAL OF MEDICINAL CHEMISTRY Bd. 33, Nr. 1 , 1990 , WASHINGTON US Seiten 52 - 60 G. BECK ET AL.

## Beschreibung

Die Erfindung betrifft neue substituierte Pyridine, Verfahren zu ihrer Herstellung, sowie ihre Verwendung in Arzneimitteln.

Es ist bekannt, daß aus Pilzkulturen isolierte Lacton-Derivate Inhibitoren der 3-Hydroxy-3-methyl-glutaryl Coenzym A Reduktase (HMG-CoA-Reduktase) sind [Mevinolin, EP 22 478; US-4 231 938].

Außerdem ist bekannt, daß Pyridin-substituierte Dihydroxyheptensäuren Inhibitoren der HMG-CoA-Reduktase sind [EP 325 130; EP 307 342; EP 306 929].

Die vorliegende Erfindung betrifft jetzt neue substituierte Pyridine der allgemeinen Formel (I) 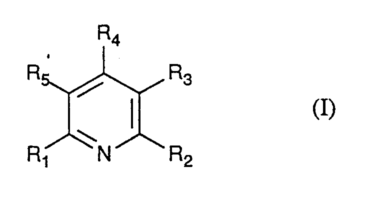 in welcher
- R¹: für Cycloalkyl mit 3 bis 7 Kohlenstoffatomen steht, oder
für geradkettiges oder verzweigtes Alkyl mit bis zu 8 Kohlenstoffatomen steht, oder
für Phenyl steht, das gegebenenfalls bis zu 2-fach gleich oder verschieden durch Halogen, Trifluormethyl oder durch geradkettiges oder verzweigtes Alkyl mit bis zu 6 Kohlenstoffatomen substituiert ist, oder
für einen Rest der Formel 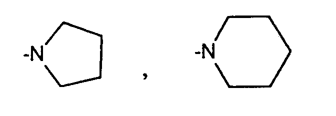 oder -NR⁶R⁷ steht,
worin
- R⁶ und R⁷: gleich oder verschieden sind und Wasserstoff oder geradkettiges oder verzweigtes Alkyl mit bis zu 4 Kohlenstoffatomen bedeuten,
- R²: für Cycloalkyl mit 3 bis 7 Kohlenstoffatomen steht, oder
für geradkettiges oder verzweigtes Alkyl mit bis zu 8 Kohlenstoffatomen steht,
- R³: für einen Rest der Formel 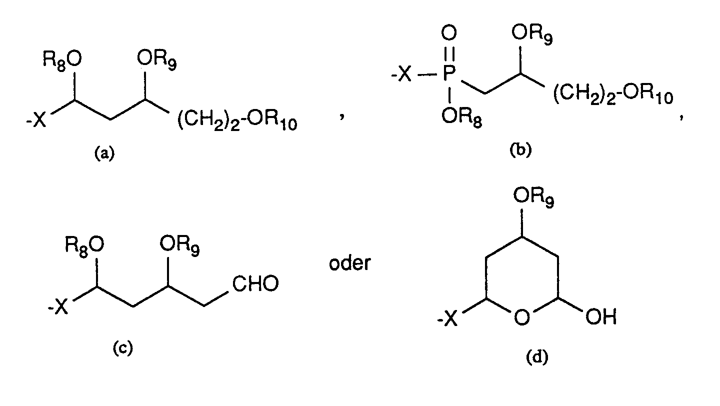 steht,
worin
- X: die Gruppe -CH₂-CH₂-, CH=H- oder -C≡C- bedeutet,
- R⁸, R⁹ und R¹⁰: gleich oder verschieden sind und Wasserstoff oder einen Rest der Formel -CO-R¹¹ oder -COO-R¹² bedeuten,
worin
- R¹¹ und R¹²: gleich oder verschieden sind und geradkettiges oder verzweigtes Alkyl mit bis zu 8 Kohlenstoffatomen oder Phenyl bedeuten,
oder
- R⁸ und R⁹: gemeinsam einen Rest der Formel 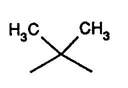 bilden,
- R⁴: für Phenyl steht, das gegebenenfalls bis zu 2-fach gleich oder verschieden durch Halogen, Trifluormethyl, Methoxy, Phenoxy oder geradkettiges oder verzweigtes Alkyl mit bis zu 8 Kohlenstoffatomen substituiert ist,
- R⁵: die oben angegebene Bedeutung von R³ hat und mit dieser gleich oder verschieden ist, oder
für Wasserstoff, Cyano, Carboxy oder geradkettiges oder verzweigtes Alkoxycarbonyl mit bis zu 8 Kohlenstoffatomen steht, oder
für geradkettiges oder verzweigtes Alkyl mit bis zu 8 Kohlenstoffatomen steht, das gegebenenfalls durch Halogen, Hydroxy oder durch geradkettiges oder verzweigtes Alkoxy mit bis zu 8 Kohlenstoffatomen oder durch eine Gruppe der Formel -O-(CH₂)ₐ-R¹³ oder -O-CO-R¹⁴ substituiert ist,
worin
- a: eine Zahl 0 oder 1 bedeutet,
- R¹³: Phenyl oder Benzyl bedeutet, die gegebenenfalls bis zu 2-fach gleich oder verschieden durch Halogen, Trifluormethyl, Cyano, Nitro oder durch geradkettiges oder verzweigtes Alkyl mit bis zu 6 Kohlenstoffatomen substituiert sind,
- R¹⁴: die oben angegebene Bedeutung von R¹³ hat und mit dieser gleich oder verschieden ist, oder
geradkettiges oder verzweigtes Alkyl mit bis zu 8 Kohlenstoffatomen bedeutet,
oder
- R⁵: für einen Rest der Formel -CH=N-O-R¹⁵ steht,
worin
- R¹⁵: die oben angegebene Bedeutung von R¹⁴ hat und mit dieser gleich oder verschieden ist.

Die erfindungsgemäßen Verbindungen haben jeweils in Abhängigkeit der unter R³ und/oder R⁵ aufgeführten Seitenketten, 1 oder 2 asymmetrische Kohlenstoffatomen, an denen die Reste -OR⁸ und -OR⁹ gebunden sind. Sie können daher in verschiedenen stereochemischen Formen existieren.

Die Erfindung betrifft sowohl die einzelnen Isomeren als auch deren Mischungen. So können die erfindungsgemäßen Stoffe je nach der relativen Stellung der Reste -OR⁸ / -OR⁹ in der Erythro-Konfiguration oder in der Threo-Konfiguration vorliegen. Dies kann für den Substituenten (a) [R³/R⁵] beispielhaft erläutert werden:

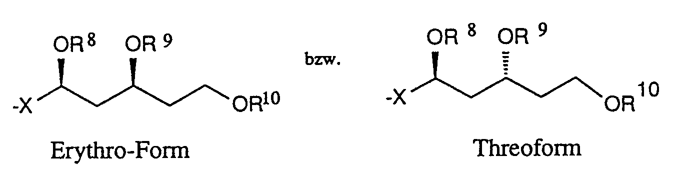

Sowohl von den Stoffen in Threo- als auch in Erythro-Konfiguration existieren wiederum jeweils zwei Enantiomere.

Darüber hinaus können die erfindungsgemäßen Stoffe aufgrund der Doppelbindung (X = -CH=CH-) in E-Konfiguration oder der Z-Konfiguration vorliegen. Bevorzugt sind diejenigen Verbindungen, die E-konfiguriert sind.

Außerdem stehen die Aldehydreste jeweils im Gleichgewicht mit den entsprechenden Pyranen

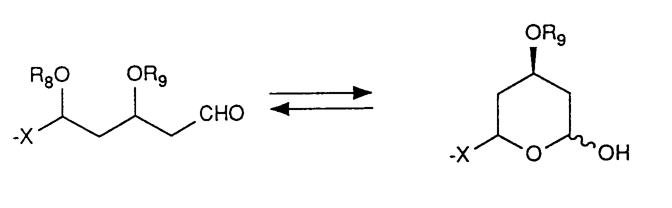

Bevorzugt sind Verbindungen der allgemeinen Formel (I)
in welcher
- R¹: für Cyclopropyl, Cyclopentyl oder für geradkettiges oder verzweigtes Alkyl mit bis zu 6 Kohlenstoffatomen steht, oder
für Phenyl steht, das gegebenenfalls durch Fluor, Chlor, Trifluormethyl oder Methyl substituiert ist,
- R²: für Cyclopropyl, Cyclopentyl oder Cyclohexyl steht, oder
für geradkettiges oder verzweigtes Alkyl mit bis zu 6 Kohlenstoffatomen steht,
- R³: für einen Rest der Formel 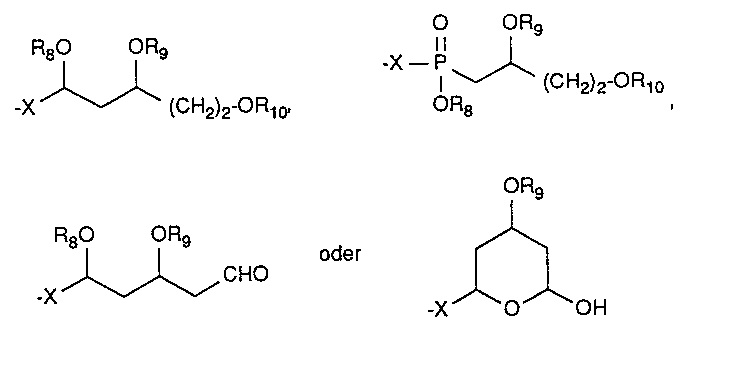 steht,
worin
- X: die Gruppe -CH₂-CH₂-, CH=H- oder -C≡C- bedeutet,
- R⁸, R⁹ und R¹⁰: gleich oder verschieden sind und Wasserstoff oder einen Rest der Formel -CO-R¹¹ oder -COO-R¹² bedeuten,
worin
- R¹¹ und R¹²: gleich oder verschieden sind und geradkettiges oder verzweigtes Alkyl mit bis zu 6 Kohlenstoffatomen oder Phenyl bedeuten,
oder
- R⁸ und R⁹: gemeinsam einen Rest der Formel 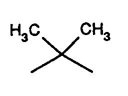 bilden,
- R⁴: für Phenyl steht, das gegebenenfalls bis zu 2-fach gleich oder verschieden durch Fluor, Chlor, Brom, Trifluormethyl oder durch geradkettiges oder verzweigtes Alkyl mit bis zu 6 Kohlenstoffatomen substituiert ist,
- R⁵: die oben angegebene Bedeutung von R³ hat und mit dieser gleich oder verschieden ist, oder
für Wasserstoff, Cyano, Carboxy oder geradkettiges oder verzweigtes Alkoxycarbonyl mit bis zu 6 Kohlenstoffatomen steht, oder
für geradkettiges oder verzweigtes Alkyl mit bis zu 6 Kohlenstoffatomen steht, das gegebenenfalls durch Fluor, Chlor, Brom, Hydroxy oder durch geradkettiges oder verzweigtes Alkoxy mit bis zu 6 Kohlenstoffatomen substituiert ist, oder das durch eine Gruppe der Formel - O-(CH₂)ₐ-R¹³ oder -O-CO-R¹⁴ substituiert ist,
worin
- a: eine Zahl 0 oder 1 bedeutet,
und
- R¹³: Phenyl oder Benzyl bedeutet, die gegebenenfalls durch Fluor, Chlor, Brom, Trifluormethyl, Cyano, Nitro oder durch geradkettiges oder verzweigtes Alkyl mit bis zu 4 Kohlenstoffatomen substituiert sind,
- R¹⁴: die oben angegebene Bedeutung von R¹³ hat und mit dieser gleich oder verschieden ist, oder geradkettiges oder verzweigtes Alkyl mit bis zu 6 Kohlenstoffatomen bedeutet,
oder
- R⁵: für einen Rest der Formel -CH=N-O-R¹⁵ steht,
worin
- R¹⁵: die oben angegebene Bedeutung von R¹⁴ hat und mit dieser gleich oder verschieden ist.

Besonders bevorzugt sind Verbindungen der allgemeinen Formel (I),
in welcher
- R¹: für Cyclopropyl oder geradkettiges oder verzweigtes Alkyl mit bis zu 4 Kohlenstoffatomen steht,
- R²: für Cyclopropyl oder für geradkettiges oder verzweigtes Alkyl mit bis zu 4 Kohlenstoffatomen steht,
- R³: für einen Rest der Formel 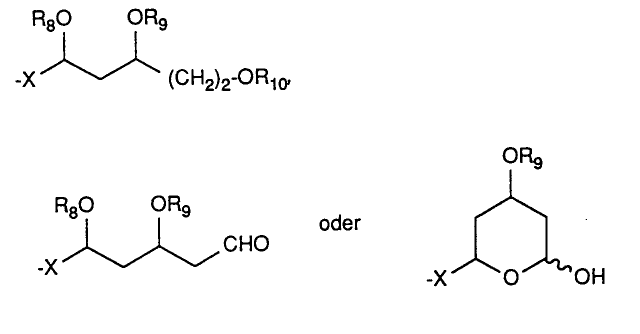 steht,
worin
- X: die Gruppe -CH₂-CH₂- oder CH=CH- bedeutet,
- R⁸, R⁹ und R¹⁰: gleich oder verschieden sind und Wasserstoff oder einen Rest der Formel -CO-R¹¹ bedeuten,
worin
- R¹¹: geradkettiges oder verzweigtes Alkyl mit bis zu 4 Kohlenstoffatomen bedeutet,
- R⁴: für Phenyl steht, das gegebenenfalls durch Fluor, Trifluormethyl oder durch geradkettiges oder verzweigtes Alkyl mit bis zu 4 Kohlenstoffatomen substituiert ist,
- R⁵: die oben angegebene Bedeutung von R³ hat und mit dieser gleich oder verschieden ist, oder
für Wasserstoff oder
für geradkettiges oder verzweigtes Alkyl mit bis zu 4 Kohlenstoffatomen steht, das gegebenenfalls durch Hydroxy oder durch geradkettiges oder verzweigtes Alkoxy mit bis zu 4 Kohlenstoffatomen oder durch p-Fluorbenzyloxy substituiert ist.

Ganz besonders bevorzugt sind Verbindungen der allgemeinen Formel (I)
in welcher
- R¹: für geradkettiges oder verzweigtes Alkyl mit bis zu 4 Kohlenstoffatomen steht,
- R²: für Cyclopropyl oder für geradkettiges oder verzweigtes Alkyl mit bis zu 4 Kohlenstoffatomen steht,
- R³: für einen Rest der Formel 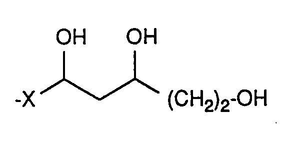 steht,
worin
- X: die Gruppe -CH₂-CH₂- oder -CH=CH- bedeutet,
- R⁴: für Phenyl steht, das gegebenenfalls durch Fluor substituiert ist,
und
- R⁵: die oben angegebene Bedeutung von R³ hat oder
für geradkettiges oder verzweigtes Alkyl mit bis zu 4 Kohlenstoffatomen steht, das gegebenenfalls durch Hydroxy oder Alkoxy mit bis zu 4 Kohlenstoffatomen oder durch Fluorbenzyloxy substituiert sein kann.

Außerdem wurde ein Verfahren zur Herstellung der erfindungsgemäßen Verbindungen der allgemeinen Formel (I) gefunden, das dadurch gekennzeichnet ist, daß man

Verbindungen der allgemeinen Formel (II) 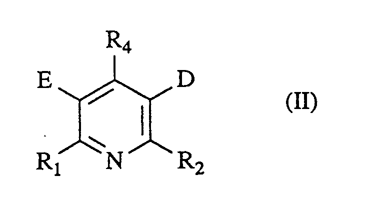 in welcher
- R¹, R² und R⁴: die oben angegebene Bedeutung haben
und
- D: für einen Rest der Formel 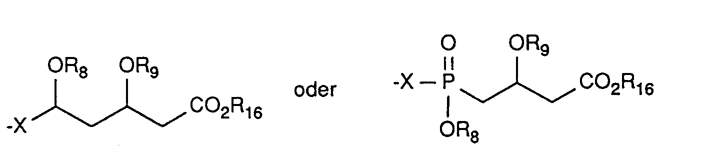 steht,
worin
- X, R⁸ und R⁹: die oben angegebene Bedeutung haben,
- R¹⁶: für C₁-C₆-Alkyl steht
und
- E: entweder ebenfalls die oben angegebene Bedeutung von D hat oder für einen unter R³ oben angegebenen anderen Substituenten steht,
in inerten Lösemitteln, unter Schutzgasatmosphäre, gegebenenfalls über die Stufe des Aldehyds reduziert,
und im Fall, daß -X- für die -CH₂-CH₂-Gruppe steht, die Ethengruppe (X = -CH=CH-) oder die Ethingruppe (X = -C≡C-) sukzessive nach üblichen Methoden hydriert, und gegebenenfalls die Isomeren trennt.

Das erfindungsgemäße Verfahren kann durch folgendes Formelschema beispielhaft erläutert werden:

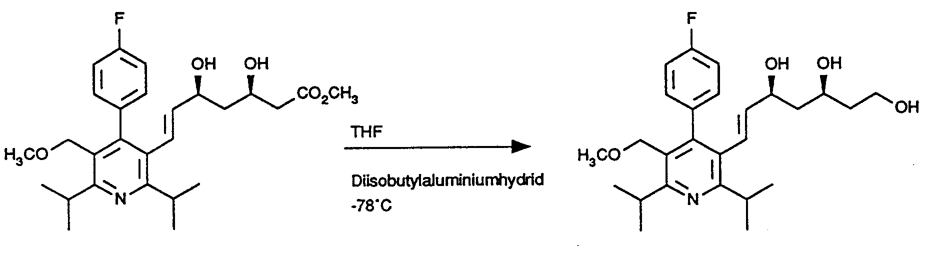

Als Lösemittel für die Reduktion eignen sich im allgemeinen die üblichen organischen Losemittel. Bevorzugt sind Ether wie Diethylether, Tetrahydrofuran oder Dioxan. Besonders bevorzugt ist Tetrahydrofuran.

Als Reduktionsmittel eignen sich komplexe Metallhydride, wie beispielsweise Lithiumalumihiumhydrid, Natriumcyanoborhydrid, Natriumaluminiumhydrid, Diisoburylaluminiumhydrid oder Natrium-bis-(2-methoxyethoxy)dihydroaluminat. Bevorzugt ist Diisobutylaluminiumhydrid.

Das Reduktionsmittel wird im allgemeinen in einer Menge von 4 mol bis 10 mol, bevorzugt von 4 mol bis 5 mol bezogen auf 1 mol der Verbindungen der allgemeinen Formel(II) eingesetzt.

Die Reduktion verläuft im allgemeinen in einem Temperaturbereich von - 78°C bis +50°C, bevorzugt von -78°C bis 0°C, besonders bevorzugt bei -78°C, jeweils in Abhängigkeit von der Wahl des Reduktionsmittels sowie Lösemittel.

Die Reduktion verläuft im allgemeinen bei Normaldruck, es ist aber auch möglich bei erhöhtem oder erniedrigtem Druck zu arbeiten.

Die Cyclisierung der Aldehyde zu den entsprechenden Pyranen erfolgt im allgemeinen bei Raumtemperatur oder durch Erhitzen in inerten organischen Lösemitteln, gegebenenfalls in Anwesenheit von Molsieb.

Als Lösemittel eignen sich hierbei Kohlenwasserstoffe wie Benzol, Toluol, Xylol, Erdölfraktionen, oder Tetralin oder Diglyme oder Triglyme. Bevorzugt werden Benzol, Toluol oder Xylol eingesetzt. Ebenso ist es möglich Gemische der genannten Lösemittel einzusetzen. Besonders bevorzugt verwendet man Kohlenwasserstoffe, insbesondere Toluol, in Anwesenheit von Molsieb.

Die Cyclisierung wird im allgemeinen in einem Temperaturbereich von - 40°C bis +100°C, bevorzugt von -25°C bis +50°C durchgeführt.

Die Hydrierung erfolgt nach üblichen Methoden mit Wasserstoff in Anwesenheit von Edelmetallkatalysatoren, wie beispielsweise Pd/C, Pt/C oder Raney-Nickel in einem der oben aufgeführten Lösemittel, vorzugsweise in Alkoholen wie beispielsweise Methanol, Ethanol oder Propanol, in einem Temperaturbereich von -20°C bis +100°C, bevorzugt von 0°C bis +50°C, bei Normaldruck oder Überdruck.

Gegebenenfalls erfolgt die Reduktion der Dreifach- bzw. Doppelbindung auch bei der oben aufgeführten Reduktion der Estergruppe.

Die Verbindungen der allgemeinen Formel (II) sind im Fall, daß D und/oder E für den Rest der Formel 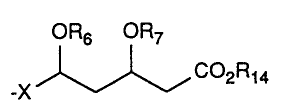 steht,
bekannt [vgl. EP 325 130].

Im Fall, daß D und/oder E für den Rest der Formel 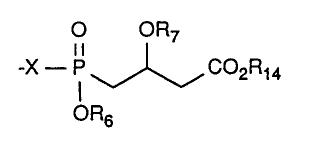 steht,
sind die Verbindungen größenteils neu. Sie können aber in Analogie zu dem in der DOS 40 23 308 publizierten Verfahren hergestellt werden, indem man ausgehend von den Verbindungen der allgemeinen Formel (III) 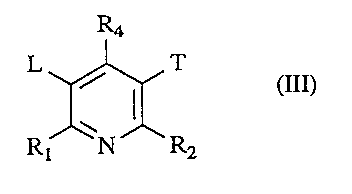 in welcher
- R¹, R² und R⁴: die oben angegebene Bedeutung haben
und entweder L und T oder L oder T für den Rest der Formel 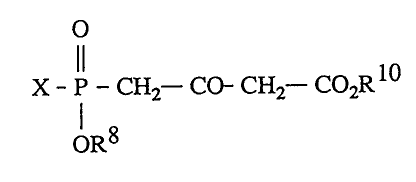 steht,
worin
- R⁸ und R¹⁰: die oben angegebene Bedeutung haben,
reduziert.

Als Losemittel eignen sich in diesem Fall insbesondere Alkohole wie Methanol, Ethanol oder Propanol, bevorzugt Ethanol.

Die Reduktion kann mit den üblichen Reduktionsmitteln, bevorzugt mit solchen, die für die Reduktion von Ketonen zu Hydroxyverbindungen geeignet sind, durchgeführt werden. Besonders geeignet ist hierbei die Reduktion mit Metallhydriden oder komplexen Metallhydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithium-trialkylhydrido-boranat oder Lithiumaluminiumhydrid durchgeführt. Ganz besonders bevorzugt wird die Reduktion mit Natriumborhydrid, in Anwesenheit von Triethylboran durchgeführt.

Die Reduktion verläuft im allgemeinen in einem Temperaturbereich von - 78°C bis +50°C, bevorzugt von -78°C bis +25°C und unter Normaldruck.

Die Verbindungen der allgemeinen Formel (III) sind größenteils neu und können in Analogie zu bekannten Methoden hergestellt werden [vgl. DE 38 172 98 A; US 50 91 378].

Im Fall der enantiomerenreinen Verbindungen der allgemeinen Formel (I) werden entweder die entsprechenden enantiomerenreinen Ester der allgemeinen Formel (II) eingesetzt, die nach publizierten Verfahren durch Umsetzung der racemischen Produkten mit enantiomerenreinen Aminen zu den entsprechenden diastereomeren Amidgemischen, anschließender Trennung durch Chromatographie oder Kristallisation in die einzelnen Diastereomere und anschließender Hydrolyse erhalten werden [vgl. DOS 40 40 026] oder man trennt die racemischen Endprodukte nach üblichen chromatographischen Methoden.

Die erfindungsgemäßen substituierten Pyridine sowie deren isomere Formen besitzen wertvolle, im Vergleich zum Stand der Technik überlegene, pharmakologische Eigenschaften; insbesondere sind sie in vivo hochwirksame Inhibitoren der 3-Hydroxy-3-methyl-glutaryl Coenzym A (HGM-CoA) Reduktase und infolge dessen Inhibitoren der Cholesterolbiosynthese. Sie können deshalb zur Behandlung von Hyperlipoproteinämie oder Arteriosklerose eingesetzt werden. Die erfindungsgemäßen Wirkstoffe bewirken außerdem eine Senkung des Cholesteringehaltes im Blut.

Die pharmakologische Wirkung der erfindungsgemäßen Stoffe wurde in folgendem Test bestimmt:

### Biologischer Test für HMGCoA-Reduktaseinhibitoren

Cholesterin wird im Säugetierorgänismus aus Acetateinheiten aufgebaut. Um die hepatische Cholesterinbiosynthese in vivo zu messen, wurde den Tieren radioaktiv markiertes ¹⁴C-Acetat appliziert und später der Gehalt an ¹⁴C-Cholesterin in der Leber bestimmt.

Die zu untersuchenden Substanzen wurden auf eine Hemmung der hepatischen Cholesterinbiosynthese in vivo an männlichen Wistar-Ratten mit einem Körpergewicht zwischen 140 und 160 g geprüft Die Ratten wurden zu diesem Zweck 18 h vor der oralen Applikation der Substanzen gewogen, in Gruppen zu 6 Tieren (Kontrollgruppe ohne Substanzbelastung 8 Tiere) eingeteilt und nüchterngesetzt. Die zu untersuchenden Subtanzen wurden direkt vor der Applikation in wäßriger 0,75 %iger Traganthsuspension mit einem Ultra-Turrax suspendiert Die Applikation der Traganthsuspension (Kontrolltiere) bzw. der in Traganth suspendierten Substanzen erfolgte mittels einer Schlundsonde. 2 h nach der oralen Substanzgabe wurden den Tieren ¹⁴C-Acetat (12,5 µCi/Tier) intraperitoneal injiziert.

Weitere 2 h später (4 h nach Substanzapplikation) wurden die Tiere durch Halsschnitt getötet und entblutet. Anschließend wurde der Bauchraum geöffnet und eine Leberprobe von ca. 700 mg zur Bestimmung des aus ¹⁴C-Acetat synthetisierten ¹⁴C-Cholesterins entnommen. Die Extraktion des Cholesterins erfolgte modifiziert nach Duncan et al. (J. Chromatogr. 162 (1979) 281-292)). Die Leberprobe wurde in einem Glaspotter in Isopropanol homogenisiert. Nach Schütteln und anschließender Zentrifugation wurde der Überstand mit alkoholischer KOH versetzt und die Cholesterinester verseift. Nach der Verseifung wurde das Gesamtcholesterin mit Heptan ausgeschüttelt und der Überstand eingedampft. Der Rückstand wurde in Isopropanol aufgenommen, in Szintillationsröhrchen überführt und mit LSC-Cocktail aufgefüllt. Das aus ¹⁴C-Acetat in der Leber synthetisierte ¹⁴C-Cholesterin wurde im Flüssigkeitsszintillationszähler gemessen. Der hepatische ¹⁴C-Cholesteringehalt, der nur mit Traganth behandelten Tiere diente als Kontrolle. Die inhibitorische Aktivität der Substanzen wird in % des synthetisierten hepatischen ¹⁴C-Cholesteringehaltes der

**Tabelle 1**

| Akute Hemmung der ¹⁴C-Cholesterinbiosynthese in vivo in der Rattenleber | |
|---|---|
| Beispiel Nr. | Dosis, die die hepatische ¹⁴C-Cholesterinsynthese zu 50 % hemmt (µg/kg KG p.o.) |
| 1 | 20 |

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,1 µg/kg bis etwa 100 µg/kg, bevorzugt in Gesamtmengen von etwa 1 µg/kg bis 50 µg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und dem Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

### Ausgangsverbindung

### Beispiel I

### 3R,5S-(+)-Methyl-erythro-(E)-7-[4-(4-fluorphenyl)-2,6-diisopropyl-5-methoxymethyl-pyrid-3-yl]-3,5-dihydroxy-hept-6-enoat

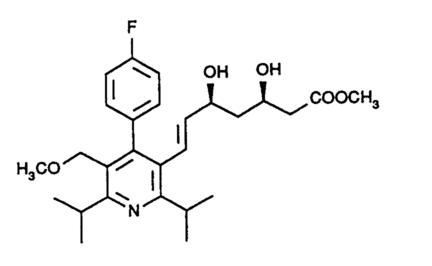

4,81 g (10 mmol) 3R,5S-(+)-Natrium-erythro-(E)-7-[4-(4-fluorphenyl)-2,6-diisopropy-5-methoxymethyl-pyrid-3-yl]-3,5-dihydroxy-hept-6-enoat werden in 100 ml Wasser gelöst und durch Zugabe von 1 N Salzsäure wird pH 4 eingestellt Es wird 2 mal mit 150 ml Methylenchlorid extrahiert Die organische Phase wird mit Na₂SO₄ getrocknet und dann bis zu vollständigen Umsetzung mit einer ätherischen Diazomethanlösung umgesetzt. Anschließend wird das Lösemittel am Rotationsverdampfer abgezogen und der Rückstand im Hochvakuum getrocknet.
Ausbeute: 4,65 g (98% d.Th.)
¹H-NMR (CDCl₃): δ = 1,23 (m, 6H); 1,32 (d, 6H); 1,40 (m, 2H); 2,43 (m, 2H); 3,18 (s, 3H); 3,32 (m, 2H); 3,73 (s, 3H); 4,05 (s, 2H); 4,08 (m, 1H); 4,29 (m, 1H); 5,23 (dd, 1H); 6,31 (d, 1H); 7,0 - 7,20 (m, 4H) ppm.
Spezifische Drehung (EtOH): [α]²⁰_{D} = 41,7° (c=1,0)

### Herstellungsbeispiele

### Beispiel 1

### 3S,5S-(+)-(E)-7-[4-(4-Fluorphenyl)-2,6-diisopropyl-5-methoxymethylpyrid-3yl]-hept-6-en-1,3,5-triol

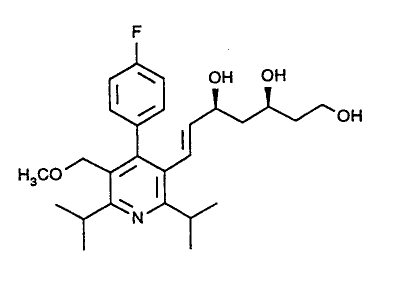

4 g (8,5 mmol) der Verbindung aus Beispiel I werden unter Argon in 100 ml absolutem THF gelöst. Bei -78°C werden 35,8 ml (43 mmol) einer 1,2 M Diisobutylaluminiumhydrid-Lösung (in Toluol) zugetropft. Nach 12 h bei - 30°C läßt man die Reaktionslösung auf 0°C kommen und gibt vorsichtig 150 ml Wasser zu. Anschließend wird 3 mal mit je 200 ml Essigester extrahiert. Die organische Phase wird mit gesättigter NaCl-Lösung gewaschen, mit Na₂SO₄ getrocknet und am Rotationsverdampfer eingeengt. Nach Chromatographie über Kieselgel 60 (25 - 40 µ, Laufmittel Essigester/Petrolether 7/3) erhält man das gewünschte Produkt.
Ausbeute: 1,98 g (52% d.Th.)
¹H-NMR (CDCl₃): δ = 1,2 - 1,5 (m, 2H); 1,23 (dd, 6H); 1,36 (d, 6H); 1,65 (m, 2H); 3,19 (s, 3H); 3,30 (m, 2H); 3,82 (m, 2H); 4,02 (m, 1H); 4,07 (s, 2H); 4,27 (m, 1H); 5,26 (dd, 1H); 6,28 (d, 1H); 7,0 - 7,2 (m, 4H) ppm.
Spezifische Drehung (EtOH): [α]²⁰_{D} = 31,1° (c=1,0)

### Beispiel 2

### 3S,5S-(+)-(E)-7-[5-(4-Fluorbenzyloxymethyl)-4-(4-fluorphenyl)-2,6-di-isopropyl-pyrid-3-yl]hept-6-en-1,3,5-triol

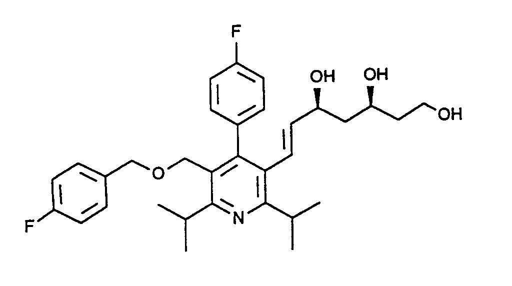

Die Herstellung erfolgt analog den Beispielen I und 1 aus 3R,5S-(+)-Natrium-erythro-(E)-7-[5-(4-fluorberzyloxymethyl)-4-(4-fluorphenyl)-2,6-diisopropyl-pyrid-3-yl]-3,5-dihydroxy-hept-6-enoat.
¹H-NMR (CDCl₃): δ = 1,1 - 1,4 (m, 2H); 1,18 (dd, 6H); 1,22 (d, 6H); 1,58 (m, 2H); 3,32 (m, 2H); 3,78 (m, 2H); 3,95 (m, 1H); 4,06 (s, 2H); 4,18 (m, 1H); 4,22 (s, 2H); 5,18 (dd, 1H); 6,22 (dd, 1H); 6,8 - 7,2 (m, 8H) ppm.

### Beispiel 3

### Erythro-(E)-7-[4-(4-fluorphenyl)-5-hydroxymethyl-2,6-diisopropyl-pyrid-3-yl]hept-6-ene-1,3,5-triol

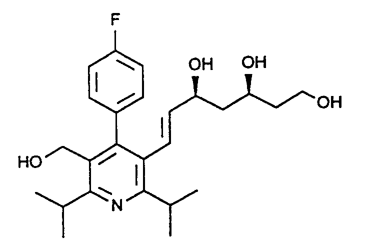

Die Herstellung erfolgt analog den Beispielen I und 1 aus Natrium-erythro-[4-(4-fluorphenyl)-5-hydroxymethyl-2,6-diisopropyl-pyrid-3-yl]-3,5-dihydroxy-hept-6-enoat.
¹H-NMR (CDCl₃): δ = 1,2 - 1,4 (m, 2H); 1,22 (dd, 6H); 1,32 (d, 6H); 1,55 (m, 2H); 3,28 (sept., 1H); 3,42 (sept., 1H); 3,86 (m, 2H); 4,03 (m, 1H); 4,28 (m, 1H); 4,42 (d, 2H); 5,27 (dd, 1H); 6,28 (d, 1H); 7,0 - 7,2 (m, 2H) ppm.

### Beispiel 4

### 2,6-Diisopropyl-4-(4-fluorphenyl)-3,5-bis-(erythro-3,5,7-trihydroxy-hept-1-(E)-enyl)-pyridin

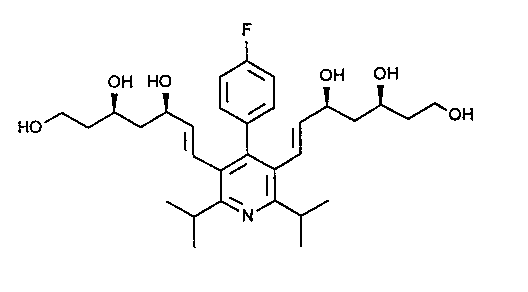

Die Herstellung erfolgt analog den Beispielen I und 1 aus 2,6-Diisopropyl-4-(4-fluorphenyl)-3,5-bis-(methyl-erythro-(E)-3,5-dihydroxy-hept-6-enoat-7-yl)-pyridin
¹H-NMR (CDCl₃): δ = 1,2 - 1,4 (m, 12H); 1,4 - 1,7 (m, 8H); 3,29 (hept. 2H); 3,83 (m, 4H); 4,03 (m, 2H); 4,30 (m, 2H); 5,28 (dd, 2H); 6,28 (d, 2H); 6,9 - 7,1 (m, 4H) ppm.

### Beispiel 5

### Erythro-(E)-7-[2-(4-fluorphenyl)-4,6-diisopropyl-5-methoxymethyl-pyrid-3-yl]hept-6-en-1,3,5-triol

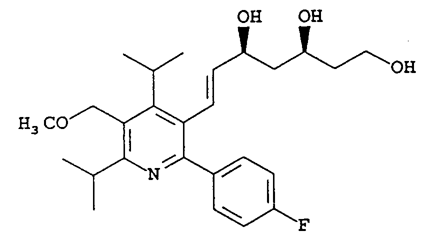

### Herstellung analog Beispiel 1 aus Methyl-erythro-(E)-7-[2-(4-fluorphenyl)-4,6-diisopropyl-5-methoxymethyl-pyrid-3-yl]-3,5-dihydroxy-hept-6-enoat

Ausbeute: 34 %, farbloses Öl
MS (DCI): 446 (30 %, M+H)

## Patentansprüche

1. Neue substituierte Pyridine der allgemeinen Formel 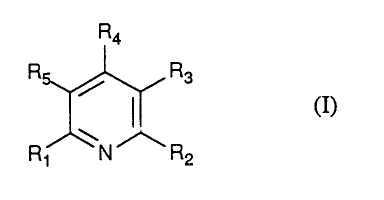 in welcher
R¹ für Cycloalkyl mit 3 bis 7 Kohlenstoffatomen steht, oder
für geradkettiges oder verzweigtes Alkyl mit bis zu 8 Kohlenstoffatomen steht, oder
für Phenyl steht, das gegebenenfalls bis zu 2-fach gleich oder verschieden durch Halogen, Trifluormethyl oder durch geradkettiges oder verzweigtes Alkyl mit bis zu 6 Kohlenstoffatomen substituiert ist, oder
für einen Rest der Formel 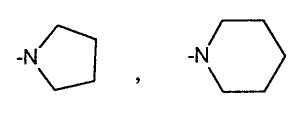 oder -NR⁶R⁷ steht,
worin
R⁶ und R⁷ gleich oder verschieden sind und Wasserstoff oder geradkettiges oder verzweigtes Alkyl mit bis zu 4 Kohlenstoffatomen bedeuten,
R² für Cycloalkyl mit 3 bis 7 Kohlenstoffatomen steht, oder
für geradkettiges oder verzweigtes Alkyl mit bis zu 8 Kohlenstoffatomen steht,
R³ für einen Rest der Formel 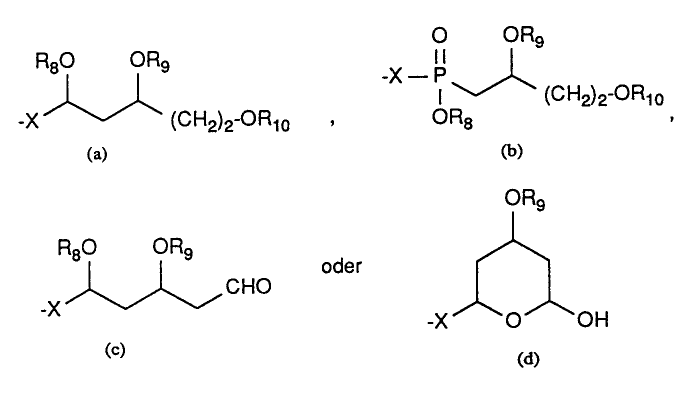 steht,
worin
X die Gruppe -CH₂-CH₂-, CH=CH- oder -C≡C-bedeutet,
R⁸, R⁹ und R¹⁰ gleich oder verschieden sind und Wasserstoff oder einen Rest der Formel -CO-R¹¹ oder -COO-R¹² bedeuten,
worin
R¹¹ und R¹² gleich oder verschieden sind und geradkettiges oder verzweigtes Alkyl mit bis zu 8 Kohlenstoffatomen oder Phenyl bedeuten,
oder
R⁸ und R⁹ gemeinsam einen Rest der Formel 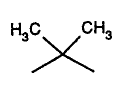 bilden,
R⁴ für Phenyl steht, das gegebenenfalls bis zu 2-fach gleich oder verschieden durch Halogen, Trifluormethyl, Methoxy, Phenoxy oder geradkettiges oder verzweigtes Alkyl mit bis zu 8 Kohlenstoffatomen substituiert ist,
R⁵ die oben angegebene Bedeutung von R³ hat und mit dieser gleich oder verschieden ist, oder
für Wasserstoff, Cyano, Carboxy oder geradkettiges oder verzweigtes Alkoxycarbonyl mit bis zu 8 Kohlenstoffatomen steht, oder
für geradkettiges oder verzweigtes Alkyl mit bis zu 8 Kohlenstoffatomen steht, das gegebenenfalls durch Halogen, Hydroxy oder durch geradkettiges oder verzweigtes Alkoxy mit bis zu 8 Kohlenstoffatomen oder durch eine Gruppe der Formel -O-(CH₂)ₐ-R¹³ oder -O-CO-R¹⁴ substituiert ist,
worin
a eine Zahl 0 oder 1 bedeutet,
R¹³ Phenyl oder Benzyl bedeutet, die gegebenenfalls bis zu 2-fach gleich oder verschieden durch Halogen, Trifluormethyl, Cyano, Nitro oder durch geradkettiges oder verzweigtes Alkyl mit bis zu 6 Kohlenstoffatomen substituiert sind,
R¹⁴ die oben angegebene Bedeutung von R¹³ hat und mit dieser gleich oder verschieden ist, oder
geradkettiges oder verzweigtes Alkyl mit bis zu 8 Kohlenstoffatomen bedeutet,
oder
R⁵ für einen Rest der Formel -CH=N-O-R¹⁵ steht,
worin
R¹⁵ die oben angegebene Bedeutung von R¹⁴ hat und mit dieser gleich oder verschieden ist.

2. Neue substituierte Pyridine nach Anspruch 1
worin
R¹ für Cyclopropyl, Cyclopentyl oder für geradkettiges oder verzweigtes Alkyl mit bis zu 6 Kohlenstoffatomen steht, oder
für Phenyl steht, das gegebenenfalls durch Fluor, Chlor, Trifluormethyl oder Methyl substituiert ist,
R² für Cyclopropyl, Cyclopentyl oder Cyclohexyl steht, oder
für geradkettiges oder verzweigtes Alkyl mit bis zu 6 Kohlenstoffatomen steht,
R³ für einen Rest der Formel 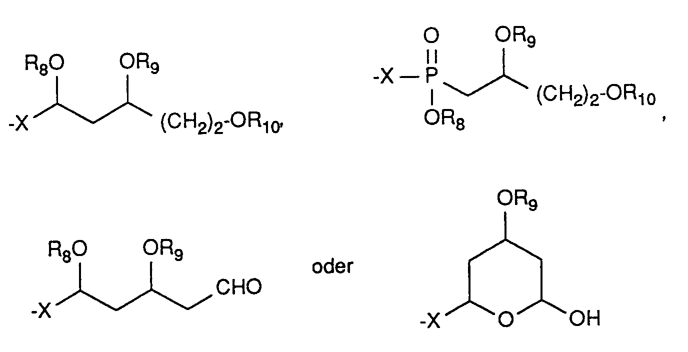 steht,
worin
X die Gruppe -CH₂-CH₂-, CH=CH- oder -C≡C-bedeutet,
R⁸, R⁹ und R¹⁰ gleich oder verschieden sind und Wasserstoff oder einen Rest der Formel -CO-R¹¹ oder -COO-R¹² bedeuten,
worin
R¹¹ und R¹² gleich oder verschieden sind und geradkettiges oder verzweigtes Alkyl mit bis zu 6 Kohlenstoffatomen oder Phenyl bedeuten,
oder
R⁸ und R⁹ gemeinsam einen Rest der Formel 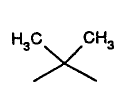 bilden,
R⁴ für Phenyl steht, das gegebenenfalls bis zu 2-fach gleich oder verschieden durch Fluor, Chlor, Brom, Trifluormethyl oder durch geradkettiges oder verzweigtes Alkyl mit bis zu 6 Kohlenstoffatomen substituiert ist,
R⁵ die oben angegebene Bedeutung von R³ hat und mit dieser gleich oder verschieden ist, oder
für Wasserstoff, Cyano, Carboxy oder geradkettiges oder verzweigtes Alkoxycarbonyl mit bis zu 6 Kohlenstoffatomen steht, oder
für geradkettiges oder verzweigtes Alkyl mit bis zu 6 Kohlenstoffatomen steht, das gegebenenfalls durch Fluor, Chlor, Brom, Hydroxy oder durch geradkettiges oder verzweigtes Alkoxy mit bis zu 6 Kohlenstoffatomen substituiert ist, oder das durch eine Gruppe der Formel -O-(CH₂)ₐ-R¹³ oder -O-CO-R¹⁴ substituiert ist,
worin
a eine Zahl 0 oder 1 bedeutet,
und
R¹³ Phenyl oder Benzyl bedeutet, die gegebenenfalls durch Fluor, Chlor, Brom, Trifluormethyl, Cyano, Nitro oder durch geradkettiges oder verzweigtes Alkyl mit bis zu 4 Kohlenstoffatomen substituiert sind,
R¹⁴ die oben angegebene Bedeutung von R¹³ hat und mit dieser gleich oder verschieden ist, oder
geradkettiges oder verzweigtes Alkyl mit bis zu 6 Kohlenstoffatomen bedeutet,
oder
R⁵ für einen Rest der Formel -CH=N-O-R¹⁵ steht,
worin
R¹⁵ die oben angegebene Bedeutung von R¹⁴ hat und mit dieser gleich oder verschieden ist.

3. Neue substituierte Pyridine nach Anspruch 1
worin
R¹ für Cyclopropyl oder geradkettiges oder verzweigtes Alkyl mit bis zu 4 Kohlenstoffatomen steht,
R² für Cyclopropyl oder für geradkettiges oder verzweigtes Alkyl mit bis zu 4 Kohlenstoffatomen steht,
R³ für einen Rest der Formel 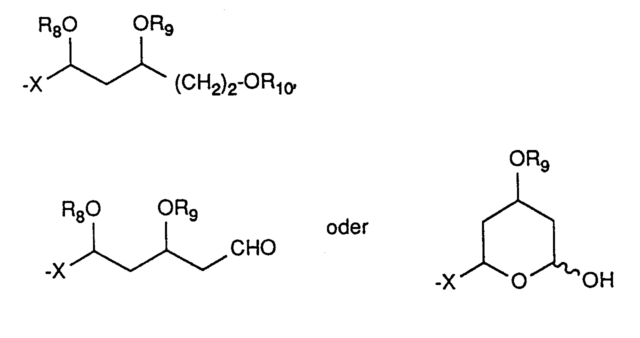 steht,
worin
X die Gruppe -CH₂-CH₂- oder CH=CH- bedeutet,
R⁸, R⁹ und R¹⁰ gleich oder verschieden sind und Wasserstoff oder einen Rest der Formel -CO-R¹¹ bedeuten,
worin
R¹¹ geradkettiges oder verzweigtes Alkyl mit bis zu 4 Kohlenstoffatomen bedeutet,
R⁴ für Phenyl steht, das gegebenenfalls durch Fluor, Trifluormethyl oder durch geradkettiges oder verzweigtes Alkyl mit bis zu 4 Kohlenstoffatomen substituiert ist,
R⁵ die oben angegebene Bedeutung von R³ hat und mit dieser gleich oder verschieden ist, oder
für Wasserstoff oder
für geradkettiges oder verzweigtes Alkyl mit bis zu 4 Kohlenstoffatomen steht, das gegebenenfalls durch Hydroxy oder durch geradkettiges oder verzweigtes Alkoxy mit bis zu 4 Kohlenstoffatomen oder durch p-Fluorbenzyloxy substituiert ist.

4. Neue substituierte Pyridine nach Anspruch 1
worin
R¹ für geradkettiges oder verzweigtes Alkyl mit bis zu 4 Kohlenstoffatomen steht,
R² für Cyclopropyl oder für geradkettiges oder verzweigtes Alkyl mit bis zu 4 Kohlenstoffatomen steht, 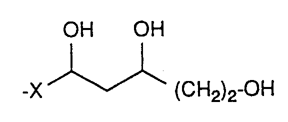 steht,
worin
X die Gruppe -CH₂-CH₂- oder -CH=CH- bedeutet,
R⁴ für Phenyl steht, das gegebenenfalls durch Fluor substituiert ist,
und
R⁵ die oben angegebene Bedeutung von R³ hat oder
für geradkettiges oder verzweigtes Alkyl mit bis zu 4 Kohlenstoffatomen steht, das gegebenenfalls durch Hydroxy oder Alkoxy mit bis zu 4 Kohlenstoffatomen oder durch p-Fluorbenzyloxy substituiert sein kann.

5. Neue substituierte Pyridine nach Anspruch 1 zur therapeutischen Anwendung.

6. Verfahren zur Herstellung von neuen substituierten Pyridinen nach Anspruch 1 dadurch gekennzeichnet, daß man
Verbindungen der allgemeinen Formel (II) 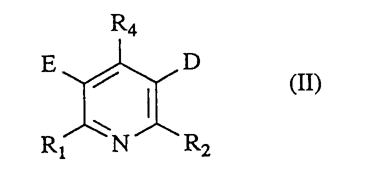 in welcher
R¹, R² und R⁴ die oben angegebene Bedeutung haben
und
D für einen Rest der Formel 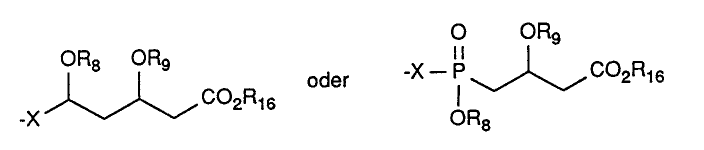 steht,
worin
X, R⁸ und R⁹ die oben angegebene Bedeutung haben,
R¹⁶ für C₁-C₆-Alkyl steht
und
E entweder ebenfalls die oben angegebene Bedeutung von D hat oder für einen unter R³ oben aufgeführten anderen Substituenten steht,
in inerten Lösemitteln, unter Schutzgasatmosphäre, gegebenenfalls über die Stufe des Aldehyds reduziert,
und im Fall, daß -X- für die -CH₂-CH₂-Gruppe steht, die Ethengruppe (X = -CH=CH-) oder die Ethingruppe (X = -C≡C-) sukzessive nach üblichen Methoden hydriert, und gegebenenfalls die Isomeren trennt.

7. Arzneimittel enthaltend mindestens ein neues substituiertes Pyridin nach Anspruch 1.

8. Arzneimittel nach Anspruch 7 zur Behandlung von Hyperlipoproteinämie.

9. Verfahren zur Herstellung von Arzneimitteln nach Anspruch 7 dadurch gekennzeichnet, daß man die neuen substituierten Pyridine gegebenenfalls mit Hilfe von üblichen Hilfs- und Trägerstoffen in eine geeignete Applikationsform überführt.

10. Verwendung von neuen substituierten Pyridinen nach Anspruch 1 zur Herstellung von Arzneimitteln.

## Claims

1. New substituted pyridines of the general formula 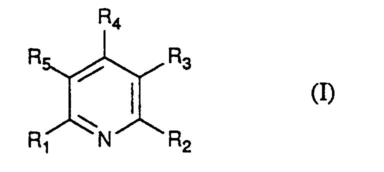 in which
R¹ represents cycloalkyl having 3 to 7 carbon atoms, or
represents straight-chain or branched alkyl having up to 8 carbon atoms, or
represents phenyl which is optionally up to disubstituted by identical or different substituents from the series consisting of halogen, trifluoromethyl or straight-chain or branched alkyl having up to 6 carbon atoms, or
represents a radical of the formula 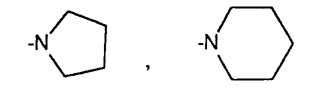 or -NR⁶R⁷,
in which
R⁶ and R⁷ are identical or different and denote hydrogen or straight-chain or branched alkyl having up to 4 carbon atoms,
R² represents cycloalkyl having 3 to 7 carbon atoms, or
represents straight-chain or branched alkyl having up to 8 carbon atoms,
R³ represents a radical of the formula 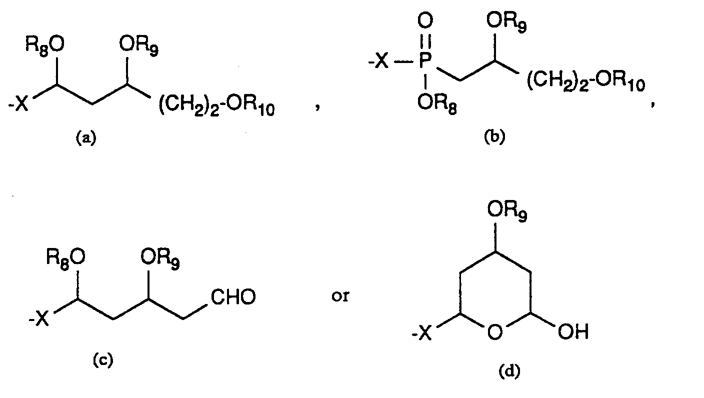 in which
X denotes the group -CH₂-CH₂-, -CH=CH- or -C≡C-,
R⁸,R⁹ and R¹⁰ are identical or different and denote hydrogen or a radical of the formula -CO-R¹¹ or -COO-R¹²,
in which
R¹¹ and R¹² are identical or different and denote straight-chain or branched alkyl having up to 8 carbon atoms, or phenyl,
or
R⁸ and R⁹ together form a radical of the formula 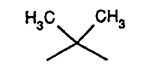
R⁴ represents phenyl which is optionally up to disubstituted by identical or different substituents from the series consisting of halogen, trifluoromethyl, methoxy, phenoxy or straight-chain or branched alkyl having up to 8 carbon atoms,
R⁵ has the abovementioned meaning of R³ and is identical with, or different from, this meaning, or
represents hydrogen, cyano, carboxyl or straight-chain or branched alkoxycarbonyl having up to 8 carbon atoms, or
represents straight-chain or branched alkyl having up to 8 carbon atoms which is optionally substituted by halogen, hydroxyl or straight-chain or branched alkoxy having up to 8 carbon atoms or by a group of the formula -O-(CH₂)ₐ-R¹³ or -O-CO-R¹⁴,
in which
a denotes a number 0 or 1,
R¹³ denotes phenyl or benzyl, each of which is optionally up to disubstituted by identical or different substituents from the series consisting of halogen, trifluoromethyl, cyano, nitro or straight-chain or branched alkyl having up to 6 carbon atoms,
R¹⁴ has the abovementioned meaning of R¹³ and is identical with, or different from, this meaning, or
denotes straight-chain or branched alkyl having up to 8 carbon atoms,
or
R⁵ represents a radical of the formula -CH=N-O-R¹⁵,
in which
R¹⁵ has the abovementioned meaning of R¹⁴ and is identical with, or different from, this meaning.

2. New substituted pyridines according to Claim 1
in which
R¹ represents cyclopropyl, cyclopentyl or straight-chain or branched alkyl having up to 6 carbon atoms, or
represents phenyl which is optionally substituted by fluorine, chlorine, trifluoromethyl or methyl,
R² represents cyclopropyl, cyclopentyl or cyclohexyl, or
represents straight-chain or branched alkyl having up to 6 carbon atoms,
R³ represents a radical of the formula 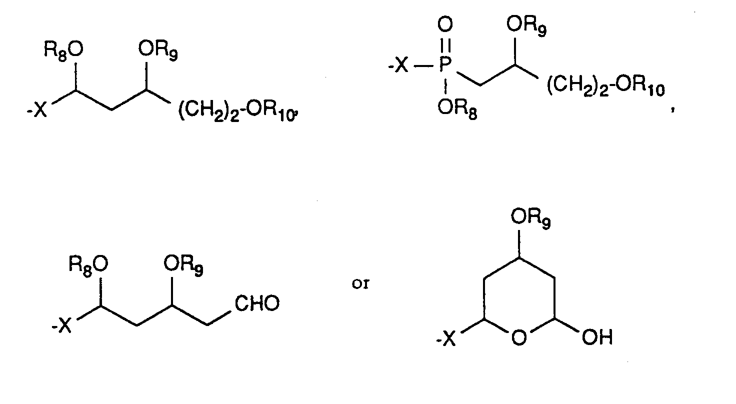 in which
X denotes the group -CH₂-CH₂-, -CH=CH- or -C≡C-,
R⁸,R⁹ and R¹⁰ are identical or different and denote hydrogen or a radical of the formula -CO-R¹¹ or -COO-R¹²,
in which
R¹¹ and R¹² are identical or different and denote straight-chain or branched alkyl having up to 6 carbon atoms, or phenyl,
or
R⁸ and R⁹ together form a radical of the formula 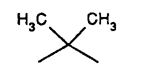
R⁴ represents phenyl which is optionally up to disubstituted by identical or different substituents from the series consisting of fluorine, chlorine, bromine, trifluoromethyl or straight-chain or branched alkyl having up to 6 carbon atoms,
R⁵ has the abovementioned meaning of R³ and is identical with, or different from, this meaning, or
represents hydrogen, cyano, carboxyl or straight-chain or branched alkoxycarbonyl having up to 6 carbon atoms, or
represents straight-chain or branched alkyl having up to 6 carbon atoms which is optionally substituted by fluorine, chlorine, bromine, hydroxyl or straight-chain or branched alkoxy having up to 6 carbon atoms, or which is substituted by a group of the formula -O-(CH₂)ₐ-R¹³ or -O-CO-R¹⁴,
in which
a denotes 0 or 1,
and
R¹³ denotes phenyl or benzyl, each of which is optionally substituted by fluorine, chlorine, bromine, trifluoromethyl, cyano, nitro or straight-chain or branched alkyl having up to 4 carbon atoms,
R¹⁴ has the abovementioned meaning of R¹³ and is identical with, or different from, this meaning, or
denotes straight-chain or branched alkyl having up to 6 carbon atoms,
or
R⁵ represents a radical of the formula -CH=N-O-R¹⁵,
in which
R¹⁵ has the abovementioned meaning of R¹⁴ and is identical with, or different from, this meaning.

3. New substituted pyridines according to Claim 1
in which
R¹ represents cyclopropyl or straight-chain or branched alkyl having up to 4 carbon atoms,
R² represents cyclopropyl or straight-chain or branched alkyl having up to 4 carbon atoms,
R³ represents a radical of the formula 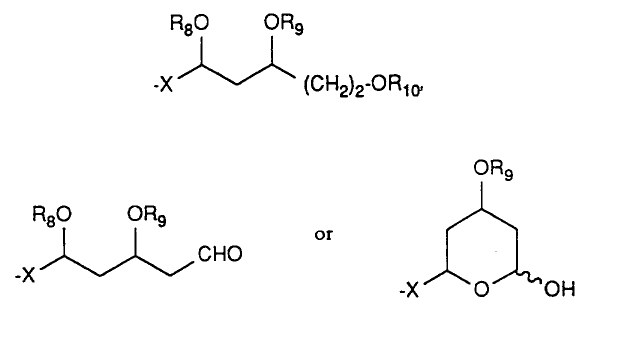 in which
X denotes the group -CH₂-CH₂- or -CH=CH-,
R⁸, R⁹ and R¹⁰ are identical or different and denote hydrogen or a radical of the formula -CO-R¹¹,
in which
R¹¹ denotes straight-chain or branched alkyl having up to 4 carbon atoms,
R⁴ represents phenyl which is optionally substituted by fluorine, trifluoromethyl or by straight-chain or branched alkyl having up to 4 carbon atoms,
R⁵ has the abovementioned meaning of R³ and is identical with, or different from, this meaning, or
represents hydrogen or
represents straight-chain or branched alkyl having up to 4 carbon atoms which is optionally substituted by hydroxyl or by straight-chain or branched alkoxy having up to 4 carbon atoms or by p-fluorobenzyloxy.

4. New substituted pyridines according to Claim 1
in which
R¹ represents straight-chain or branched alkyl having up to 4 carbon atoms,
R² represents cyclopropyl or straight-chain or branched alkyl having up to 4 carbon atoms, 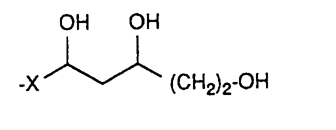 in which
X denotes the group -CH₂-CH₂- or -CH=CH-,
R⁴ represents phenyl which is optionally substituted by fluorine,
and
R⁵ has the abovementioned meaning of R³ or
represents straight-chain or branched alkyl having up to 4 carbon atoms which can optionally be substituted by hydroxyl or alkoxy having up to 4 carbon atoms or by p-fluorobenzyloxy.

5. New substituted pyridines according to Claim 1 for use in therapy.

6. Process for the preparation of new substituted pyridines according to Claim 1, characterised in that
compounds of the general formula (II) 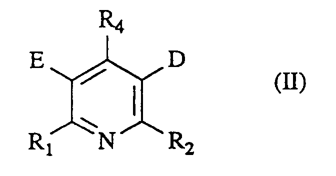 in which
R¹, R² and R⁴ have the abovementioned meaning
and
D represents a radical of the formula 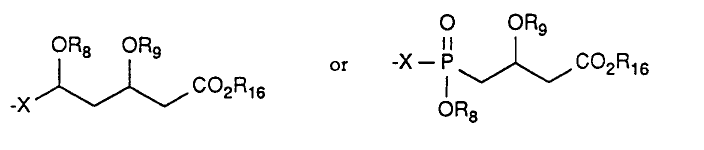 in which
X, R⁸ and R⁹ have the abovementioned meaning,
R¹⁶ represents C₁-C₆-alkyl
and
E either also has the abovementioned meaning of D or represents a different substituent mentioned above under R³,
are reduced in inert solvents under a protective gas atmosphere, if appropriate via the aldehyde stage,
and, in the event that -X- represents the -CH₂-CH₂-group, the ethene group (X = -CH=CH-) or the ethyne group (X = -C≡C-), are hydrogenated gradually by customary methods and, if appropriate, the isomers are separated.

7. Pharmaceutical comprising at least one new substituted pyridine according to Claim 1.

8. Pharmaceutical according to Claim 7 for the treatment of hyperlipoproteinaemia.

9. Process for the preparation of pharmaceuticals according to Claim 7, characterised in that the new substituted pyridines are brought into a suitable form for administration, if appropriate with the aid of customary auxiliaries and carriers.

10. Use of new substituted pyridines according to Claim 1 for the preparation of pharmaceuticals.

## Revendications

1. Nouvelles pyridines substituées répondant à la formule générale 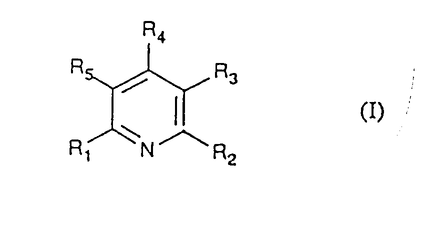 dans laquelle
R¹ représente un groupe cycloalkyle contenant de 3 à 7 atomes de carbone, ou
représente un groupe alkyle à chaîne droite ou ramifiée contenant jusqu'à 8 atomes de carbone, ou encore
représente un groupe phényle qui porte éventuellement jusqu'à 2 substituants identiques ou différents halogéno, trifluorométhyle ou alkyle à chaîne droite ou ramifiée contenant jusqu'à 6 atomes de carbone,
ou encore représente un radical répondant aux formules 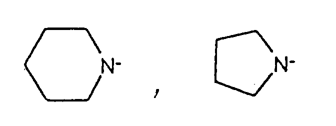 ou -NR⁶R⁷
où
R⁶ et R⁷ sont identiques ou différents et représentent un atome d'hydrogène ou encore un groupe alkyle à chaîne droite ou ramifiée contenant jusqu'à 4 atomes de carbone,
R² représente un groupe cycloalkyle contenant de 3 à 7 atomes de carbone, ou
représente un groupe alkyle à chaîne droite ou ramifiée contenant jusqu'à 8 atomes de carbone,
R³ représente un radical répondant aux formules 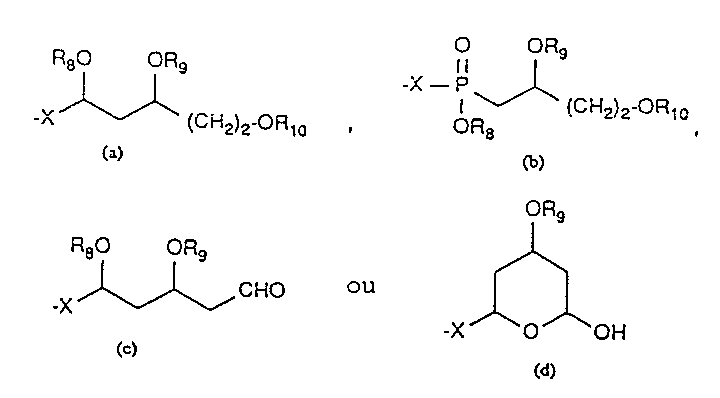 dans lesquelles
X représente le groupe -CH₂-CH₂-, CH=CH- ou -C≡C-,
R⁸, R⁹ et R¹⁰ sont identiques ou différents et représentent un atome d'hydrogène ou un radical de formule -CO-R¹¹ ou -COO-R¹²,
dans lesquelles
R¹¹ et R¹² sont identiques ou différents et représentent un groupe alkyle à chaîne droite ou ramifiée contenant jusqu'à 8 atomes de carbone ou encore un groupe phényle,
ou bien
R⁸ et R⁹ forment ensemble un radical de formule 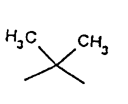
R⁴ représente un groupe phényle qui porte éventuellement jusqu'à 2 substituants identiques ou différents halogéno, trifluorométhyle, méthoxy, phénoxy ou alkyle à chaîne droite ou ramifiée contenant jusqu'à 8 atomes de carbone,
R⁵ a la signification de R³ indiquée ci-dessus et est identique à ou différent de ce dernier, ou bien
représente un atome d'hydrogène, un groupe cyano, un groupe carboxyle ou un groupe alcoxycarbonyle à chaîne droite ou ramifiée contenant jusqu'à 8 atomes de carbone, ou
représente un groupe alkyle à chaîne droite ou ramifiée contenant jusqu'à 8 atomes de carbone, qui porte éventuellement un ou plusieurs substituants identiques ou différents halogéno, hydroxyle ou alcoxy à chaîne droite ou ramifiée contenant jusqu'à 8 atomes de carbone ou encore -O-(CH₂)ₐ-R¹³ ou -O-CO-R¹⁴,
dans lesquels
a représente le nombre 0 ou 1,
R¹³ représente un groupe phényle ou un groupe benzyle qui portent éventuellement jusqu'à 2 substituants identiques ou différents halogéno, trifluorométhyle, cyano, nitro ou alkyle à chaîne droite ou ramifiée contenant jusqu'à 6 atomes de carbone,
R¹⁴ possède la signification de R¹³ indiquée ci-dessus et est identique à ou différent de ce dernier, ou représente un groupe alkyle à chaîne droite ou ramifiée contenant jusqu'à 8 atomes de carbone,
ou bien
R⁵ représente un radical de formule -CH=N-O-R¹⁵
dans laquelle
R¹⁵ a la signification de R¹⁴ indiquée ci-dessus et est identique à ou différent de ce dernier.

2. Nouvelles pyridines substituées selon la revendication 1,
dans lesquelles
R¹ représente un groupe cyclopropyle, un groupe cyclopentyle ou un groupe alkyle à chaîne droite ou ramifiée contenant jusqu'à 6 atomes de carbone, ou
représente un groupe phényle qui porte éventuellement un ou plusieurs substituants identiques ou différents fluoro, chloro, trifluorométhyle ou méthyle,
R² représente un groupe cyclopropyle, un groupe cyclopentyle ou un groupe cyclohexyle, ou
représente un groupe alkyle à chaîne droite ou ramifiée contenant jusqu'à 6 atomes de carbone,
R³ représente un radical répondant aux formules 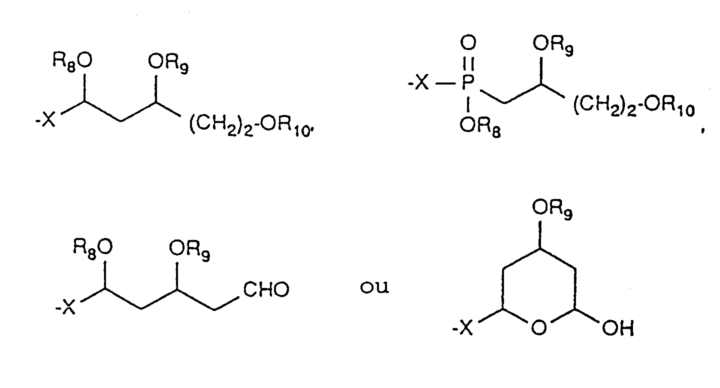 dans lesquelles
X représente le groupe -CH₂-CH₂-, CH=CH- ou -C≡C-,
R⁸, R⁹ et R¹⁰ sont identiques ou différents et représentent un atome d'hydrogène ou un radical de formule -CO-R¹¹ ou -COO-R¹²,
dans lesquelles
R¹¹ et R¹² sont identiques ou différents et représentent un groupe alkyle à chaîne droite ou ramifiée contenant jusqu'à 6 atomes de carbone ou encore un groupe phényle,
ou bien
R⁸ et R⁹ forment ensemble un radical de formule 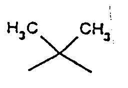
R⁴ représente un groupe phényle qui porte éventuellement jusqu'à 2 substituants identiques ou différents fluoro, chloro, bromo, trifluorométhyle ou alkyle à chaîne droite ou ramifiée contenant jusqu'à 6 atomes de carbone,
R⁵ a la signification de R³ indiquée ci-dessus et est identique à ou différent de ce dernier, ou bien
représente un atome d'hydrogène, un groupe cyano, un groupe carboxyle ou un groupe alcoxycarbonyle à chaîne droite ou ramifiée contenant jusqu'à 6 atomes de carbone, ou
représente un groupe alkyle à chaîne droite ou ramifiée contenant jusqu'à 6 atomes de carbone, qui porte éventuellement un ou plusieurs substituants identiques ou différents fluoro, chloro, bromo, hydroxyle ou alcoxy à chaîne droite ou ramifiée contenant jusqu'à 6 atomes de carbone ou encore -O-(CH₂)ₐ-R¹³ ou -O-CO-R¹⁴,
dans lesquels
a représente le nombre 0 ou 1,
R¹³ représente un groupe phényle ou un groupe benzyle qui portent éventuellement un ou plusieurs substituants identiques ou différents fluoro, chloro, bromo, trifluorométhyle, cyano, nitro ou alkyle à chaîne droite ou ramifiée contenant jusqu'à 4 atomes de carbone,
R¹⁴ possède la signification de R¹³ indiquée ci-dessus et est identique à ou différent de ce dernier, ou représente un groupe alkyle à chaîne droite ou ramifiée contenant jusqu'à 6 atomes de carbone,
ou bien
R⁵ représente un radical de formule -CH=N-O-R¹⁵
dans laquelle
R¹⁵ a la signification de R¹⁴ indiquée ci-dessus et est identique à ou différent de ce dernier.

3. Nouvelles pyridines substituées selon la revendication 1,
dans lesquelles
R¹ représente un groupe cyclopropyle ou un groupe alkyle à chaîne droite ou ramifiée contenant jusqu'à 4 atomes de carbone,
R² représente un groupe cyclopropyle ou un groupe alkyle à chaîne droite ou ramifiée contenant jusqu'à 4 atomes de carbone,
R³ représente un radical répondant aux formules 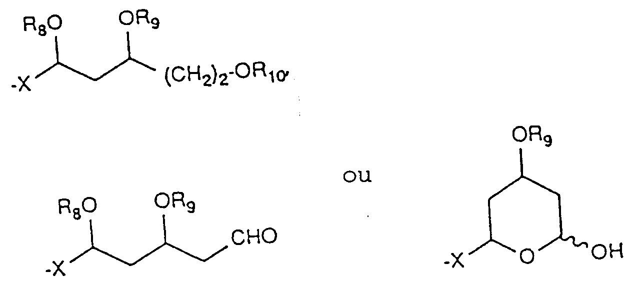 dans lesquelles
X représente le groupe -CH₂-CH₂- ou CH=CH-,
R⁸, R⁹ et R¹⁰ sont identiques ou différents et représentent un atome d'hydrogène ou un radical de formule -CO-R¹¹,
dans laquelle
R¹¹ représente un groupe alkyle à chaîne droite ou ramifiée contenant jusqu'à 4 atomes de carbone,
R⁴ représente un groupe phényle qui porte éventuellement un ou plusieurs substituants identiques ou différents fluoro, trifluorométhyle ou alkyle à chaîne droite ou ramifiée contenant jusqu'à 4 atomes de carbone,
R⁵ a la signification de R³ indiquée ci-dessus et est identique à ou différent de ce dernier, ou
représente un atome d'hydrogène, ou
représente un groupe alkyle à chaîne droite ou ramifiée contenant jusqu'à 4 atomes de carbone, qui porte éventuellement un ou plusieurs substituants identiques ou différents hydroxyle ou alcoxy à chaîne droite ou ramifiée contenant jusqu'à 4 atomes de carbone, ou encore p-fluorobenzyloxy.

4. Nouvelles pyridines substituées selon la revendication 1,
dans lesquelles
R¹ représente un groupe alkyle à chaîne droite ou ramifiée contenant jusqu'à 4 atomes de carbone,
R² représente un groupe cyclopropyle ou un groupe alkyle à chaîne droite ou ramifiée contenant jusqu'à 4 atomes de carbone, 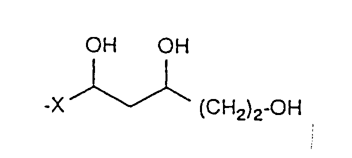 dans laquelle
X représente le groupe -CH₂-CH₂- ou -CH=CH-,
R⁴ représente un groupe phényle qui porte éventuellement un ou plusieurs substituants fluoro,
et
R⁵ a la signification de R³ indiquée ci-dessus ou
représente un groupe alkyle à chaîne droite ou ramifiée contenant jusqu'à 4 atomes de carbone, qui peut éventuellement porter un ou plusieurs substituants identiques ou différents hydroxyle ou alcoxy contenant jusqu'à 4 atomes de carbone ou encore p-fluorobenzyloxy.

5. Nouvelles pyridines substituées selon la revendication 1, pour l'utilisation thérapeutique.

6. Procédé pour préparer de nouvelles pyridines substituées selon la revendication 1, caractérisé en ce qu'on réduit des composés répondant à la formule générale (II) 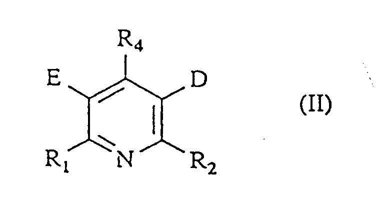 dans laquelle
R¹, R² et R⁴ ont la signification indiquée ci-dessus,
et
D représente un radical répondant aux formules 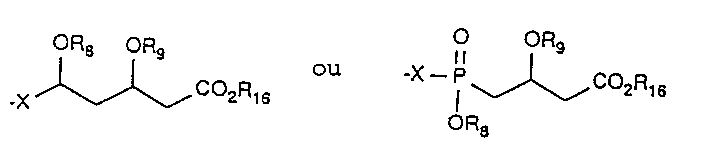 dans lesquelles
X, R⁸ et R⁹ ont la signification indiquée ci-dessus,
R¹⁶ représente un groupe alkyle en C₁-C₆
et
E soit possède également la signification de D indiquée ci-dessus, soit représente un autre substituant indiqué ci-dessus sous R³,
dans des solvants inertes, sous l'atmosphère d'un gaz de protection, éventuellement en passant par l'aldéhyde,
et dans le cas où X représente le groupe -CH₂-CH₂-, on procède à une hydrogénation, conformément à des procédés habituels, du groupe éthène (X = -CH=CH-) ou du groupe éthyne (X = -C≡C-) successivement et on sépare éventuellement les isomères.

7. Médicament contenant au moins une nouvelle pyridine substituée selon la revendication 1.

8. Médicament selon la revendication 7, pour le traitement de l'hyperlipoprotéinémie.

9. Procédé pour la préparation de médicaments selon la revendication 7, caractérisé en ce qu'on transforme les nouvelles pyridines substituées éventuellement à l'aide d'adjuvants et de substances de support habituelles en une forme d'application appropriée.

10. Utilisation de nouvelles pyridines substituées selon la revendication 1, pour la préparation de médicaments.
